Europäisches Patentamt

European Patent Office

Office européen des brevets

(11) Publication number: **0 657 502 A1**

## EUROPEAN PATENT APPLICATION

(21) Application number: **93120042.2**

(22) Date of filing: **13.12.93**

(51) Int. Cl.6: **C08L 67/02**, C08L 77/00, C08L 75/04

(43) Date of publication of application:
**14.06.95 Bulletin 95/24**

(84) Designated Contracting States:
**GB**

(71) Applicant: **DU PONT DE NEMOURS INTERNATIONAL S.A.**
**2, chemin du Pavillon,**
**P.O. Box 50**
**CH-1218 Le Grand-Saconnex (Genève) (CH)**

(72) Inventor: **Hausmann, Karl-Heinz**
**Sablons 57**
**CH-2000 Neuchâtel (CH)**
Inventor: **Cardinal, Jean-Claude**
**3, route de Courte-Raye**
**CH-1297 Founex/VD (CH)**

(54) **Thermoplastic composition containing compatibilizer.**

(57) A thermoplastic composition containing a mixture of (a) a block copolyether ester, a block copolyether amide and/or a polyurethane; (b) a thermoplastic homo- co- or terpolymer that is incompatible with (a); and (c) a compatibilizer; and its use in preparing films is disclosed. Such films demonstrate moisture vapor permeability, while acting as barriers to liquids and microorganisms.

EP 0 657 502 A1

This invention relates to a thermoplastic composition and its use in preparing films demonstrating moisture vapor permeability, while acting as barriers to liquids and microorganisms such as viruses and bacteria. Such films are found in various articles including wound coverings, transdermal patches, operating room drapes, protective clothing, diapers, personal hygiene products (feminine hygiene, incontinency), waterproof and outdoor clothing articles, food packaging, films used in plant growing environments and any end-use where it is desirable to combine "breathability" and liquid barrier properties.

Background of the Invention

Various thermoplastic materials are known for forming films demonstrating both liquid barrier properties and moisture vapor permeability. Included among these are copolyether esters, copolyether amides and polyurethanes. While films of these materials perform well and meet the performance requirements of various end uses, a drawbrack they possess is their relative high cost in comparison to other thermoplastic materials.

It is an object of the present invention to provide a new thermoplastic composition and film therefrom possessing desirable performance characteristics at a reduced cost. According to the invention, a thermoplastic composition is provided containing a mixture of (a) a block copolyether ester, a block copolyether amide and/or a polyurethane; (b) a thermoplastic homo- co- or terpolymer that is incompatible with (a); and (c) a compatibilizer.

Component (b) of the composition is a lower cost thermoplastic material which has insufficient moisture vapor permeability to be suitable for the end-uses previously mentioned. It is incompatible with component (a) and, were the two to be combined together and formed into a film, such film would have a profile showing long laminae or sheets of component (b). Accordingly, films made from such a composition would have a permeability restricted by the specific permeability of component (b), and poor mechanical properties, e.g. a tendency to delaminate.

According to the present invention, it has been surprisingly found that a compatibilizer can be combined with components (a) and (b) to yield a film having a moisture vapor transmission rate (MVTR) approaching that of the theoretical value calculated as follows:

$$\text{MVTR theor} = (\text{MVTR}_{comp.(a)})(\text{weight \%}_{comp.(a)}) + (\text{MVTR}_{comp.(b)})(\text{weight \%}_{comp.(b)}).$$

Detailed Description of the Invention

As used herein, certain terms are defined as follows.

"Compatibility" of thermoplastic materials is an art-recognised term that refers, generally, to the degree in which the thermoplastic materials are miscible and/or interact with each other. Accordingly, "incompatible", as used herein, means that the components (a) and (b) are substantially immiscible and/or do interact with each other.

"Compatibilizer" means a thermoplastic material which serves to prevent formation of the previously mentioned laminae of component (b) in a film according to the invention. The compatibilizer has a character which makes it simultaneously soluble or reactive with component (a) and interactive with component (b), thereby reducing the surface energy of component (b) in component (a), leading to the formation of a dispersion of globules of component (b) in the film.

Block copolyether esters, block copolyether amides and polyurethanes to be used for component (a) are known per se. Preferred block copolyether esters are segmented elastomers having soft polyether segments and hard polyester segments (c.f. U.S. Patent No. 4,739,012), available from the DuPont Company under the name Hytrel®.

Suitable block copolyether amides for use in the invention are available under the name Pebax® from Elf Atochem.

The amount of component (a) in the composition will vary depending upon the type of component (a), the type of component (b), desired level of moisture vapor permeability and other factors known to one skilled in the art. Component (a) is typically present in the composition according to the invention in an amount ranging from 25 to 90 % by weight, more preferably 50 to 80 %.

Component (b) according to the invention, is, as previously mentioned, substantially incompatible with component (a). Component (b) may be a homopolymer of an alpha-olefin; a co- or terpolymer containing an alpha-olefin and one or more other monomers; or a block copolymer of a vinylarene and a conjugated diene.

Where component (b) is a homopolymer, it preferably contains the repeating unit $R\text{-}CH = CH_2$ in which R is hydrogen or an alkyl radical having between 1 and 8 carbon atoms. Preferred homopolymers according to the invention are polyethylene and polypropylene.

Where component (b) is a co- or terpolymer, it preferably contains the repeating unit $R\text{-}CH = CH_2$ above, with at least one further monoethylenically unsaturated, such as aliphatic or aromatic, monomer, the following of which can be cited by way of example: vinyl acetate, styrene, and (meth)acrylic derivatives. This other monomer can represent up to 20% by weight of the olefinic copolymer, preferably from 1 to 10% by weight.

Preferred copolymers to be used as component (b) according to the invention are copolymers of ethylene and propylene, ethylene-vinyl acetate copolymers, copolymers of ethylene and acrylic derivatives (e.g. copolymers of ethylene, carbon monoxide and n-butyl acrylate, commonly known as EnBACO), copolymers of ethylenically unsaturated carboxylic acid monomers (e.g. acrylic acid, methacrylic acid, crotonic acid, etc.) or the neutralized metalic salts thereof (e.g. as found in the partially neutralized ethylene/carboxylic acid copolymers which are commonly referred to in the art as ionomers), terpolymers based on olefin, methyl acrylate and ethyl acrylate or even mixtures of straight-chain and radicalar low density polyolefins.

Where component (b) is a block copolymer of of a vinylarene and a conjugated diene, it may have the general structure A-B-A wherein the two terminal polymer blocks A comprise thermoplastic polymer blocks of vinylarenes such as polystyrene, while block B is a polymer block of selectively hydrogenated conjugated diene such as isoprene or butadiene (cf. U.S. Patent Re. No. 27,145; U.S. Patent No. 4,548,988). The proportion of the thermoplastic terminal blocks to the center elastomeric polymer block and the relative molecular weights of each of these blocks is balanced to obtain a rubber having an optimum combination of properties such that it behaves as a vulcanized rubber without requiring the actual step of vulcanization. Moreover, these block copolymers can be designed not only with this important advantage but also so as to be handled in thermoplastic forming equipment and are soluable in a variety of relatively low cost solvents.

Optionally, these block copolymers can be grafted with maleic anhydride so as to form adducts which contain 0.1 to 10% by weight, preferably 0.2 to 5%, of maleic anhydride (see U.S. Patent 4,578,429). Such compounds are commonly referred to as S-EB-S block copolymers and are available from Shell Chemical Company under the name Kraton®.

Component (b) is typically present in the composition of the instant invention in a an amount ranging from 10-70% by weight.

The compatibilizer is chosen according to the nature of component (b). It will have a backbone that is compatible with, and is preferably identical to, component (b) and a reactive group which is compatible or interacts with component (a). This changes the morphology of the composition according to the invention such that the component (b) is distributed in component (b) in the form of globules rather than laminae.

The reactive group may be grafted to this backbone using a grafting monomer having at least one alpha- or beta-ethylenically unsaturated carboxylic acids and anhydrides, and derivatives thereof. Examples of such carboxylic acids and anhydrides, which may be mono-, di- or polycarboxylic acids, are acrylic acid, methacrylic acid, maleic acid, fumaric acid, itaconic acid, crotonic acid, itaconic anhydride, maleic anhydride and substituted maleic anhydride e.g. dimethyl maleic anhydride. Examples of derivatives of the unsaturated acids are salts, amides, imides and esters e.g. mono- and disodium maleate, acrylamide, maleimide and diethyl fumarate.

Maleic anhydride is a preferred grafting monomer.

The grafting of the polymers can be carried out in the melt state, in solution or in suspension as described. The melt viscosity of the grafted polymer is not restricted, however, most effective alloying is found if the melt index, measured at 2.16 kg and 190°C is between 1 and 150 $g/cm^3$. Such grafted polymers can be prepared as known in the art (cf. European Patent Application 370 735 and 370 736).

The compatibilizer is typically present in the composition of the instant invention in a an amount ranging from 0.1 to 15% by weight, preferably between 0.1 and 3%.

In addition to the above components, compositions according to the invention may contain conventional additives, such as melt flow modifying agents (e.g. calcium carbonate, silicas, clay, talc) and stabilizers, such as antioxidants and ultraviolet absorbers. The properties these additives bring to the composition and the amounts in which they can be used are known to the skilled artisan.

The compositions according to the invention can be prepared according to methods and techniques known in the art, e.g. by compounding or (physical) tumble blending.

A suitable compounding technique is as follows: a polyether ester and polyethylene are compounded in a Berstorff 25 mm twin screw extruder at a temperature ranging from 200-240 °C. Strands of copolymer so produced are water cooled and granulated.

EP 0 657 502 A1

Similarly, the compositions can be made into films using standard extrusion techniques, e.g. extrusion coating and blown film processing. For example, granules produced as above are fed into a Brabender plasticoder extruder having a length/diameter = 24 and equipped with a blown film unit. Processing temperature is from 200-220 °C. Films so produced have a thickness of about 50 $\mu$m.

Examples

Physical blends and compounded blends are prepared having the compositions indicated in Table 1 below. Films having a thickness of 25 $\mu$m are extruded and moisture vapor transmission rate (MTVR) is measured according to ASTM E 96 BW.

Polymer A = a block polyether ester segmented elastomer containing about, by weight, 31% dimethyl terephthalate, 9% dimethyl isophthalate, 55% ethylene oxide end-capped polypropylene glycol and 16% butane diol; melt flow index (MFI) = 10 dg/min at 190 °C under 2.16 kg according to ASTM D 1238).

Polymer B = Stamylan® 2304 (available from DSM; a low density polyethylene, density of 0.92 kg/m$^3$, MFI = 4.4 dg/min (ASTM D 1238)).

Polymer C = Fusabond® EMB 226 D (available from the DuPont Company; backbone of PE-LLD, density of 0.92 kg/m$^3$, MFI = 4.4 dg/min (ASTM D 1238), comonomer content: 7% butene, maleic anhydride content: 1%).

Table 1

|  |  | Control 1 | Control 2 | Control 3 | Example 1 | Example 2 |
|---|---|---|---|---|---|---|
| Polymer A | (parts by weight) | 100 |  | 70 | 65.8 | 65.8 |
| Polymer B |  |  | 100 | 30 | 28.2 | 28.2 |
| Polymer C |  |  |  |  | 6 | 6 |
| Blend type |  | - | - | physical | physical | compound |
| MTVR (g/m$^2$•day) |  | 2025 | 9 | 253 | 735 | 657 |

Films according to the invention demonstrate improved performance properties, such as improved seal strength. This can be demonstrated by sealing the film using a Kopp sealing machine with two sealing bars; dwell time 1 sec, pressure 0.3 MPa; and testing the seal on a Zwick tensile testing machine with 100 mm/min. cross head speed.

**Claims**

1. A thermoplastic composition comprising
   (a) a block copolyether ester, a block copolyether amide, and/or a polyurethane;
   (b) a thermoplastic homo- co- or terpolymer that is incompatible with (a); and
   (c) a compatibilizer.

2. A composition according to claim 1 wherein (b) is a homopolymer of an alpha-olefin; a co- or terpolymer containing an alpha-olefin and one or more other monomers; or a block copolymer of a vinylarene and a conjugated diene.

3. A composition according to claim 1 or 2 wherein (c) is a homo- co- or terpolymer whose backbone corresponds to (b) and which is grafted with a monomer having a functional group that is compatible with (a).

4. A composition according to claim 1 wherein (a) is a block copolyether ester, (b) is polyethylene, and (c) has a grafted polymer having a backbone of polyethylene that is grafted with maleic anhydride.

5. A composition according to any one of claims 1 to comprising, by weight, 25 to 90 % (a), 10 to 70% (b) and 0.1 to 15% (c).

6. A film formable from a composition according to any one of claims 1 to 5.

4

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-4 410 482 (P.M.SUBRAMANIAN)<br>* claims 1,6-8; examples 9,11 *<br>--- | 1-3,5 | C08L67/02<br>C08L77/00<br>C08L75/04 |
| A | EP-A-0 518 517 (MORTON INT., INC.)<br>* page 5, line 28 - line 33; claims 1,7,8,10 *<br>--- | 1-4 | |
| A | WO-A-91 05008 (EXXON CHEMICAL PATENTS INC.)<br>* claims 1,5,6,14 *<br>----- | 1-3 | |

TECHNICAL FIELDS
SEARCHED     (Int.Cl.6)

C08L

The present search report has been drawn up for all claims

| Place of search | Date of completion of the search | Examiner |
|---|---|---|
| BERLIN | 6 May 1994 | Angiolini, D |

EPO FORM 1503 03.82 (P04C01)